(19) Europäisches Patentamt
European Patent Office
Office européen des brevets

(11) Publication number: 0 216 162
A2

# (12) EUROPEAN PATENT APPLICATION

(21) Application number: 86111670.5

(22) Date of filing: 22.08.86

(51) Int.Cl.⁴: C 07 D 209/18
A 61 K 31/405

(30) Priority: 23.08.85 DK 3840/85

(43) Date of publication of application:
01.04.87 Bulletin 87/14

(84) Designated Contracting States:
AT BE CH DE FR GB IT LI LU NL SE

(71) Applicant: AKTIESELSKABET DE DANSKE SUKKERFABRIKKER
Langebrogade 5
DK-1001 Copenhagen K(DK)

(72) Inventor: Marcussen, Jan
35, 5, Njalsgade
DK-2300 Copenhagen S(DK)

(72) Inventor: Ulvskov, Peter Bjarne
14, Gersonsvej
DK-2900 Hellerup(DK)

(74) Representative: Vossius & Partner
Siebertstrasse 4 P.O. Box 86 07 67
D-8000 München 86(DE)

(54) **A method of preparing indoles or indole derivatives, coupled via position 4, 5, 6, or 7, the indoles or indole derivatives concerned, as well as the use thereof.**

(57) Described is a method of preparing indoles or indole derivatives, coupled via position 4, 5, 6, or 7, the indoles or indole derivatives concerned, as well as the use thereof in the preparation of biologically active affinity matrixes.

EP 0 216 162 A2

VOSSIUS & PARTNER

PATENTANWÄLTE

EUROPEAN PATENT ATTORNEYS

0216162

Dr. VOLKER VOSSIUS, Dipl.-Chem.
DOROTHEA VOSSIUS, Dipl.-Chem.
Dr. PAUL TAUCHNER, Dipl.-Chem.
Dr. DIETER HEUNEMANN, Dipl.-Phys.
Dr. PETER RAUH, Dipl.-Chem.
Dr. GERHARD HERMANN, Dipl.-Phys.

SIEBERTSTRASSE 4
P.O.BOX 860767
8000 MÜNCHEN 86
PHONE: (089) 474075
CABLE: BENZOLPATENT MÜNCHEN
TELEX: 529453 VOPAT D
TELEFAX: (089) 472001 (GR. II + III)

Our ref.: U 817 EP

August 22, 1986

Case: 56853 Er/Ve

Aktieselskabet De Danske Sukkerfabrikker
Copenhagen, Denmark

# A METHOD OF PREPARING INDOLES OR INDOLE DERIVATIVES, COUPLED VIA POSITION 4, 5, 6, or 7, THE INDOLES OR INDOLE DERIVATIVES CONCERNED, AS WELL AS THE USE THEREOF.

## Field of the Invention

The present invention relates to a novel method of preparing indoles or indole derivatives coupled via position 4, 5, 6, or 7, the novel indoles or indole derivatives as well as the use thereof.

## Description of the Prior Art

Many indole compounds are biologically active. They may either be substances of natural occurence or purely synthetic derivatives. It is important in clinical and plant physiological connections in biological systems in vivo and in vitro that the concentrations of these compounds can be determined extremely precisely, and that standardized analytical methods can be developed for these compounds.

During recent years the number of published analytical methods based on immuno-chemical reactions has increased considerably. Among the most important reasons therefor can be mentioned that these methods possess a high sensitivity and specificity at the same time as the methods have a high capacity, which makes it possible to perform many analyses a day. By utilizing monoclonal instead of polyclonal antibodies it is usually possible, by a suitable selection of the antibody producing hybridoma cells, to increase the antibody specificity and thereby minimize undesired cross reaction with related compounds. The use of monoclonal antibodies furthermore offers the great advantage that these analytical methods may be standardized. This facilitates comparisons of analytical results from different laboratories.

Indole compounds are low-molecular substances. In immunologic context these compounds function as haptenes and are thus not directly immunogenic. To obtain an immune response it is thus necessary to couple the haptene covalently to a high molecular weight carrier molecule, typically a protein. Thereby the original structure of the haptene is changed, and the similarity to the free haptene is reduced. This fact will be reflected in the specificity of the produced antibodies to the free haptene and in the cross reaction of the antibodies with related compounds.

It generally applies that the antibody specificity is slightest to the sites on the haptene molecule where the covalent bond is introduced.

This problem can be illustrated by comparing two types of antibodies against the important phytohormone, the auxine indole-3-acetic acid (IAA) having the formula

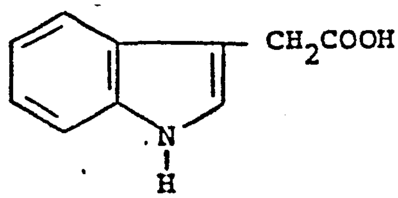

indole-3-acetic acid.

Descriptions of two methods of preparing immunogenic IAA-protein conjugates can be found in the literature. One method is based on a Mannich-reaction between the indole nitrogen atom and a protein amino group. The method was used by N.S. Ranadive and A.H. Sehon, Canadian Journal of Biochemistry, 1967, volume 45, p. 1701-1710, in connection with the preparation of immunogenic 5-hydroxytryptamin-protein conjugates and later by W. Pengelly and F. Jr. Meinz, Planta, 1977, volume 136, p. 173-180, for preparing immunogenic IAA-N-1-protein conjugates. The presumed structure of this conjugate is shown by the formula

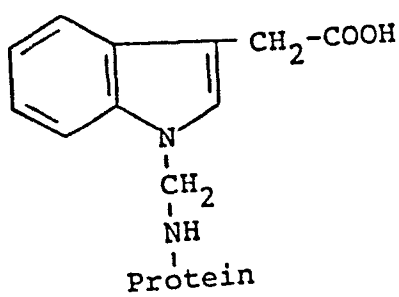

IAA-N-1-protein.

The second method is based on the introduction of an amide bond between the carboxylic acid group

and a protein amino group. N.S. Ranadive and A.H. Sehon, Canadian Journal of Biochemistry, 1967, volume 45, p. 1681-1688, utilized such immunogenic indole-protein conjugates, prepared by a symmetrical anhydride reaction between 5-hydroxyindole-3-acetic acid and a protein, to produce antibodies against the free haptene 5-hydroxyindole3-acetic acid. E.W. Weiler, Planta, 1981, volume 153, p. 319-325, used a mixed anhydride reaction between indole-3-acetic acid and a protein to introduce a similar amide bond between the ligand and a protein amino group. The structure of this conjugate is shown by the formula

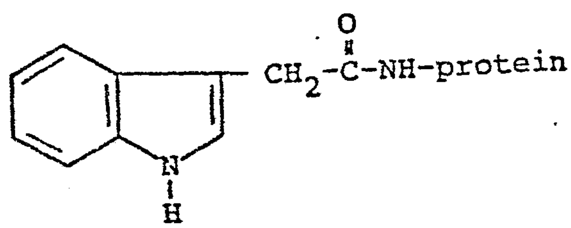

IAA-C-1$^1$-protein.

By comparing antibodies produced against the two types of IAA-protein conjugates a significant difference is observed with respect to the cross reaction of the antibodies with related compounds. Antibodies produced against IAA-N-1-protein conjugates are extremely specific to the indoleC-3-substitution, and consequently only a minor cross reaction with related indole-C-3-compounds is observed. The indole specificity is on the other hand small, and a considerable degree of cross reaction with other cyclic acetic acid derivatives, e.g. naphthalene-1-acetic acid is observed. Antibodies produced against IAA-C-$1^1$-protein conjugates show the opposite pattern of specificity. Thus a considerably higher degree of cross reaction with the other C-3-substituted indoles is observed than with antibodies produced against IAA-N-1-protein conjugates. The indole specificity of these antibodies is, however, considerably higher compared to antibodies produced against IAA-N-1-protein conjugates, and the degree of cross reaction with e.g. naphthalene-1-acetic acid is consequently considerably lower.

These results show that the indole nitrogen atom as well as the carboxylic acid group are part of the immunogenic determinant of the haptene. The results clearly show that the antibody specificity is reduced against the functional group being masked by the covalent bond between haptene and carrier molecule.

In connection with the preparation of an immunogenic haptene conjugate it is important to obtain a high degree of substitution of the chosen carrier molecule. Many indoles are labile. It is thus important that all reactions take place under mild conditions. It is also important that the coupling between haptene and carrier molecule takes place in a solvent system compatible with the stability of the carrier molecule. This is especially important when a labile or functional protein, e.g. an enzyme is used as carrier molecule. Coupling by means of activated esters in purely aqueous systems or systems containing water miscible organic solvents is suitably performed during in this step.

Summery of the Invention

The object of the present invention is to provide a general method, according to which it is possible to prepare indole carrier conjugates, where indole-N-1, indole-C-2 or indole-C-3 substituents, if any, appear in the optimum analogous form to the free indole compound concerned so that the substituents as defined below are maintained in unchanged, active form.

Consequently, the present invention relates to a method of preparing indoles or indole derivatives coupled via position 4, 5, 6, or 7, said method being characterised in reacting an indole compound hydroxyl substituted in position 4, 5, 6, or 7 of the formula II

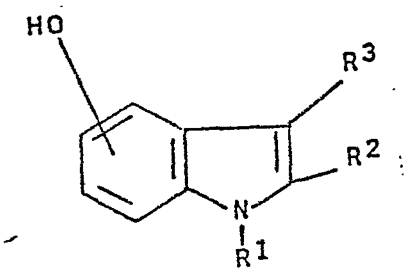

in which $R^1$, $R^2$, and $R^3$ each designates H or any other group not interfering with the subsequent steps, which groups may, if necessary, be protected by protective groups known per se, or 2 or all of the substituents $R^1$, $R^2$, and $R^3$ together with the atoms, to which they are connected, form a cyclic or heterocyclic system, with an activating reagent, and then or simultaneously introducing a nucleophilic group, optionally converting the resulting compound into an activated derivative thereof, such as an activated ester or a tosyl-,

tresyl- or other derivative, which may be a part of a nucleophilic substitution reaction, or temporarily introducing this as a part of a leaving group to bind the indole hydroxyl group covalently to the functional group, which is to be coupled thereto, via a coupling residue.

The present invention relates more specifically to a method of preparing compounds having the general formula I:

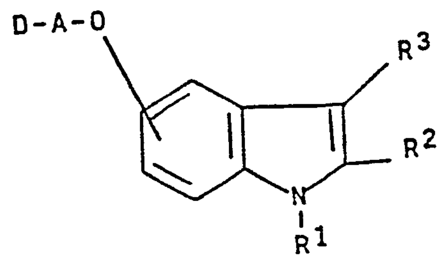

in which D is a group of the formula Y-X-D' containing a nucleophilic radical, in which X is a direct bond or a mono- or poly-substituted, straight chained or branched, saturated or unsaturated hydrocarbon group with 1-9 carbon atoms, D' is NH-, -S-, -O- or -O-Ph-, where Ph means phenyl, and Y is a carboxylic acid group or an activated ester group thereof, or Y is an alcohol group or a tosyl or tresyl derivative thereof or is temporarily introduced as a part of a leaving group and A is a coupling residue such as

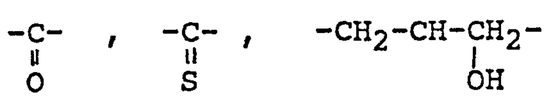 , , or $-(CH_2)_m$,

in which m is an integer from 1 to 9, and $R^1$, $R^2$, and $R^3$ have the meanings as indicated above,

said method being specific by the fact that the indole structure of formula II as defined above is reacted with a reagent, which provides the coupling residue A, and then or simultaneously introduces a nucleophilic group D, as defined above.

The method according to the present invention is of a general nature. It is thus possible to bind the carrier molecule covalently to the hydroxy group in the indole derivative in position 4, 5, 6, or 7 from the corresponding optionally substituted hydroxy indole compound. Hydroxy indoles are particularly advantageous starting materials, as they are relatively stable, and a great deal of them are commercially available.

The above objects are completely fulfilled by the present invention. By introducing a covalent bond between C-4, C-5, C-6, or C-7 on the indole structure and the carrier molecule the desired optimum analogy is obtained between the indole carrier conjugate and the free indole compound with respect to N-1, C-2 or C-3 substituents, if any, and with respect to the heterocyclic indole core itself. The indole compounds prepared according to the invention and activated in position 4, 5, 6, or 7 make it possible to control the degree of substitution of the carrier molecule.

By varying the amount of the dosed activated indole compound in connection with the method according to the invention a quantitative substitution of the carrier structure concerned can be obtained. In this connection it has been considered important

to develop methods making it possible to perform the coupling reaction under mild conditions.

Description of the Preferred Embodiments

In a preferred embodiment of the method according to the invention the activation of the hydroxyl substituted indole structure is performed with a coupling reagent chosen among carbonyl diimidazole, carbonyl thiodiimidazole or a epihalohydrine to obtain compounds, in which A means

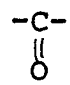 , 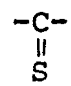 or 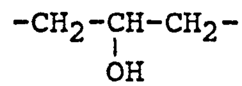

respectively.

The result of using one of these coupling reagents is that the successive reaction can take place under conditions, in which biologically significant indoles and indole analogs are stable.

In a particularly preferred embodiment of the method according to the invention a compound containing a thiol-, amino-, phenol- or alcohol group is used as a nucleophilic reagent thus allowing the introduction of charged or uncharged (protonisable or non-protonisable) groups into the indole structure.

By another preferred method according to the invention the indole or indole derivative is reacted with a substituted $C_{1-9}$-alkyl halogenide, whereby simultaneously a coupling residue A and a nucleophilic group D, in which A and D have the above indicated meanings, are introduced.

When carbonyl diimidazole or carbonyl thiodiimidazole is used as coupling reagent in the method according to the invention, the activated indole structure is in a particularly preferred embodiment of the method according to the invention washed after coupling with a buffer at a pH-value below or about neutral pH. . Excess coupling reagent is thereby destroyed, whereby the possibility of undesired side reactions are minimized. A certain purification of the activated indole structure is also achieved by this treatment.

In a particularly preferred embodiment of the method according to the invention a compound providing a carboxylic acid group is used as nucleophilic reagent, whereby a group allowing further derivatization, especially coupling, is obtained.

Nucleophilic groups containing a carboxylic acid group are advantageously introduced by means of an amino acid as the nucleophilic reagent, it hereby being possible to introduce biologically active groups into the structure, viz. an amide group.

In a particularly advantageous embodiment of the method according to the invention the above carboxylic acid group is converted to an activated ester group, whereby further coupling can take place under mild conditions, which is important to a coupling to labile or functional proteins.

In a further advantageous embodiment of the method according to the invention, the indole or indole

derivative is reacted with 3-bromo propane-1-tetrahydrofuranylether.

A further purpose of this invention is to provide novel indole-carrier-conjugates, in which indole-N-1, indole-C-2 or indole-C-3 substituents, if any, are present in optimally analogous form with the free indole compound concerned so that the substituents as defined below are maintained in unchanged, active form.

The present invention further relates to novel indoles and indole derivatives coupled via positions 4, 5, 6, or 7 characterised by the general formula I

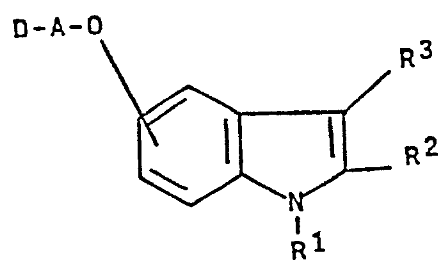

in which D-A-O is in position 4, 5, 6, or 7, and in which $R^1$, $R^2$, and $R^3$ each designates H or any other group not interfering with the subsequent steps, which groups are, if necessary, protected by protective groups known per se, or 2 or all of the substituents $R^1$, $R^2$, and $R^3$ together with the atoms, to which they are connected form part of a cyclic or heterocyclic system,

A is a coupling residue, and

D is a group of the formula Y-X-D' containing a

nucleophilic radical, in which X is a direct bond or a mono- or poly-substituted, straight chained or branched, saturated or unsaturated hydrocarbon group with 1-9 carbon atoms, D' is NH-, -S-, -O- or -O-Ph-, where Ph means phenyl, and Y is a carboxylic acid group or an activated ester group thereof, or Y is an alcohol group or a tosyl or tresyl derivative thereof or is temporarily introduced as part of a leaving group.

Preferred indoles or indole derivatives according to the invention are the compounds, in which A is a group having the formula

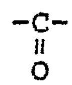 or 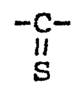

Alternatively preferred indoles or indole derivatives according to the invention are the compounds, in which A is a group having the formula

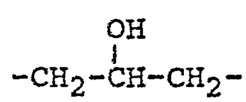

Furthermore preferred indoles or indole derivatives according to the invention are the compounds, in which A is a groups having the formula

$$(CH_2)_m$$

in which m is an integer from 1 to 9.

Particularly preferred indoles or indole derivatives are compounds having the formula

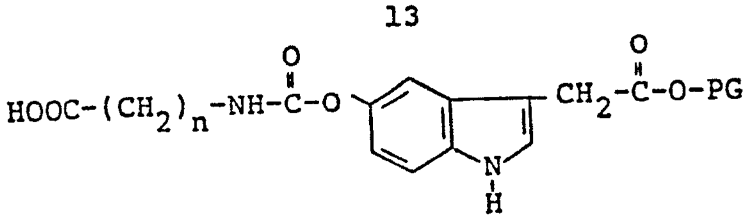

(III)

in which n is 0-9 and PG is a protective group as stated in e.g.: Theodora W. Greene: " Protective Groups in Organic Synthesis", John Wiley and Sons, Inc., New York, 1981. PG may therefore be e.g. $-CH_3$, $-CH_2CH_3$ or $-CH_2COOCH_3$.

Other preferred indoles or indole derivatives are compounds having the formula

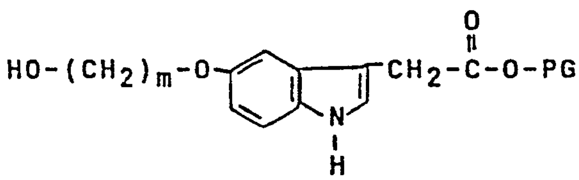

(IV)

in which m and PG have the meanings indicated above.

Further preferred indoles or indole derivatives are compounds of the formula

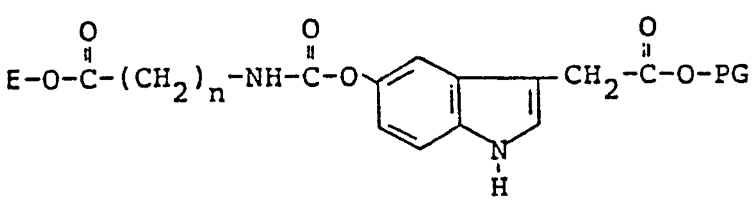

(V)

in which n and PG have the meanings indicated above and -COOE is an activated ester group.

A particularly preferred indole derivative according to the invention is the compound having the formula

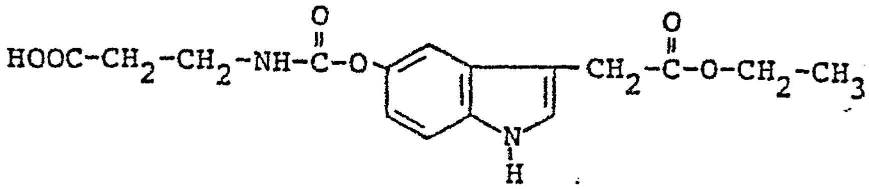 (VI)

Another particularly preferred indole derivative according to the invention is the compound having the formula

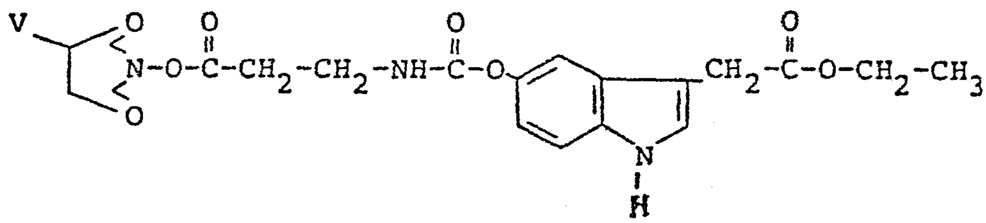 (VII)

in which V is H or $-SO_3H$ or a salt thereof, especially preferred an alkalimetal salt, such as a Li, Na or K salt.

A further particularly preferred indole derivative according to the invention is the compound of the formula

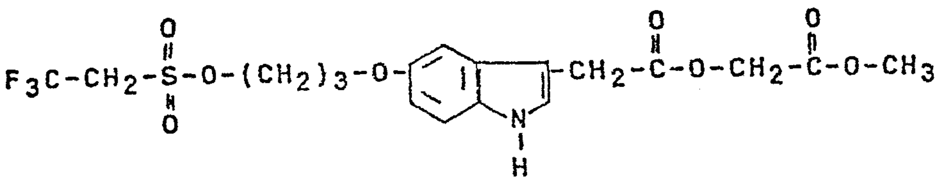 (VIII),

The above derivatives of the formulae (VI), (VII), and (VIII) are particularly preferred embodiments according to the invention making the preparation of a ligand with a high structural resemblance to the important phytohormone, the auxine indole-3-acetic acid, possible.

The present invention further relates to the use of indoles or indole derivatives according to the invention in the preparation of indole-carrier-conjugates, particularly preferred are conjugates, in which the carrier is a protein. As a result it is possible to prepare antigen or an enzyme tracer with an optimum resemblance or similarity to the free phytohormone, the auxine indole-3-acetic acid.

The invention further relates to the use of the indoles and indole derivatives of the formula (I) as previously defined, especially preferred the indole derivatives of the formulae (VI), (VII), or (VIII) in the preparation of an affinity matrix. As a result an affinity matrix is obtained for the purification of the above antibodies corresponding to the said antigens or for the purification of biologically important hormone receptors.

It will be possible to use the method here described of preparing indole derivatives as a general method of preparing ring-coupled conjugates.
The invention can thus be used in the preparation of immunogenic indole-carrier-conjugates in connection with the production of antibodies and in the preparation of enzyme tracers useful in analytical context. These indole derivatives can further be used in the preparation of affinity gels in connection with the purification of indole specific antibodies and biologically important indole receptors.

Example 1

Synthesis of 5-(N-(3-sulphosuccinimidoxycarbonylethyl) carbamoyl)-oxy-indolyl-3-acetic acid ethylester, Na-salt having the formula (4)

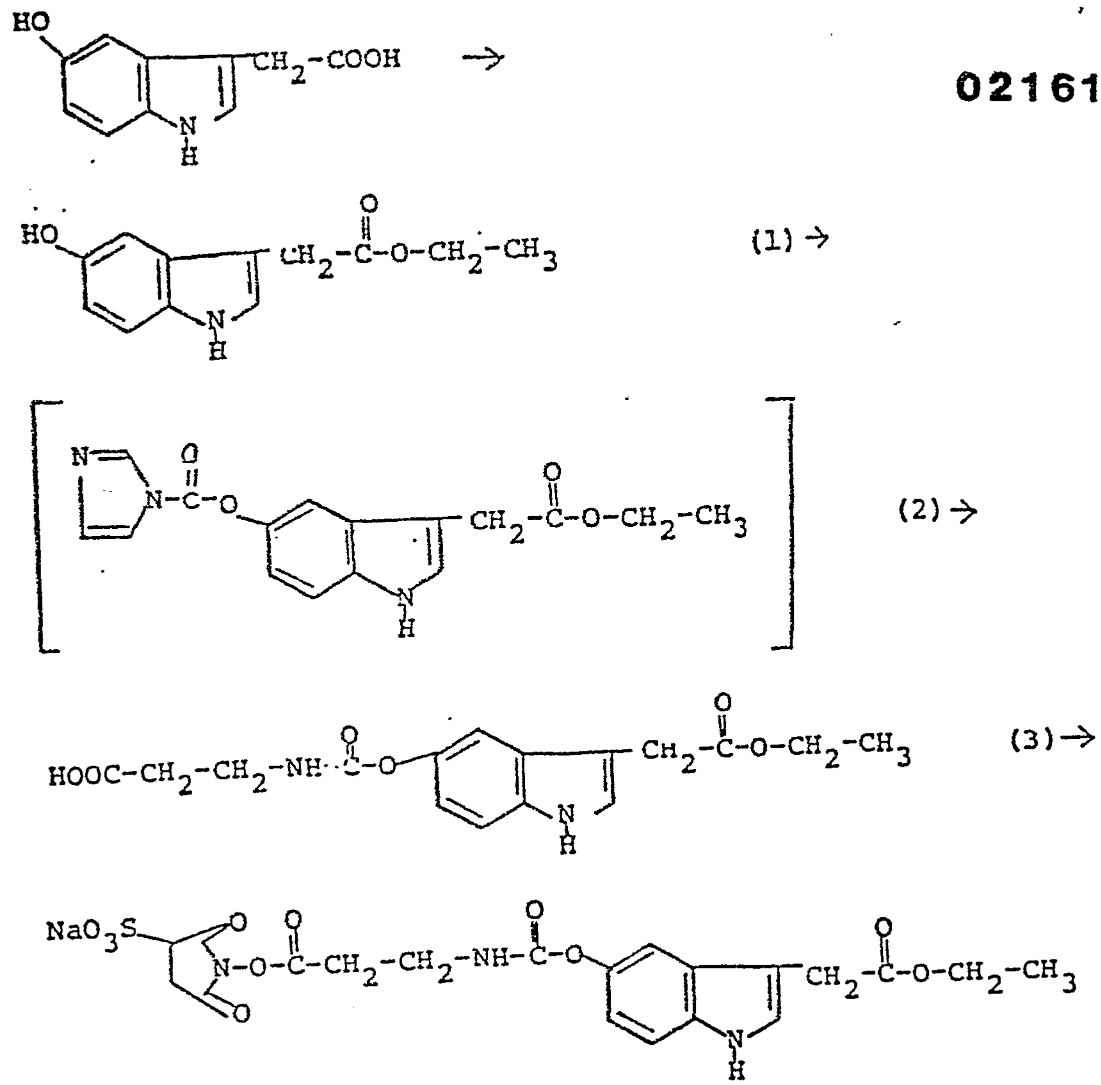

(1)

(2)

(3)

(4)

5-hydroxy-indolyl-3-acetic acid ethylester having the formula (1) 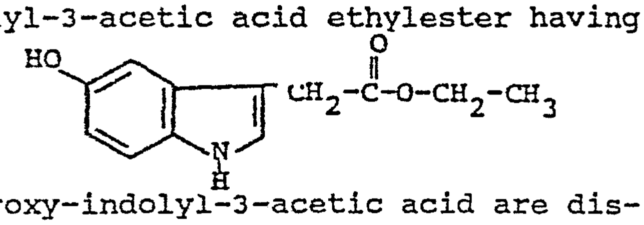

5 mmoles 5-hydroxy-indolyl-3-acetic acid are dissolved in 100 ml absolute ethanol, and 0.5 ml concentrated 37% HCl is added. The reaction mixture is closed under Ar and is left under stiring for 24 hours at room temperature. Hereafter the volume is reduced to about 50 ml by means of a rotary evaporator at 40°C, and $H_2O$ is added, until the reaction mixture gets milky. After freezing to -20°C and thawing to room temperature (1) is isolated in crystalline form.

$^1$H-nmr. (90 MHz) $\delta$ 7.2 (dd, 1H), 7.04 (d, 1H), 6.9 (dd, 1H), 6.68 (dd, 1H), 4.07 (q, J = 7.3 Hz, 2H),

3.63 (d(allylic), 2H), 2.89 (OH), 2.85 (NH), 1.20 (t, J = 7.3 Hz, 3H).
Mass spectrum m/e 146 (base peak), 219.

5-(N-(carboxyethyl)carbamoyl)-oxy-indolyl-3-acetic acid ethylester of the formula (3)

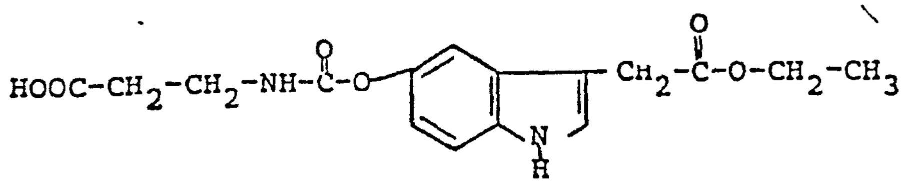

5 mmoles (1) are dissolved in 10 ml dry dioxane, and 10 mmoles of carbonyl diimidazole are added. The reaction mixture is closed under Ar and is stirred for an hour. Hereafter the solvent is removed in a rotary evaporator at 40°C. The evaporation residue containing (2) is washed with cold 0.1 M sodium phosphate buffer, pH 7.0 and immediately reacted with 7.5 mmoles 3-amino propanoic acid, dissolved in 10 ml 50% $H_2O$-dioxane, pH 8.0. The reaction mixture is closed under Ar and stirred for 24 hours. Hereafter the volume is reduced to about 2 ml on a rotary evaporator at 40°C, and 10 ml ethanol are added. After cooling the precipitates formed are filtered off. The filtrate is purified by ion exchange chromatography on QAE-Sephadex R G-25 (Pharmacia Fine Chemicals) in 50% $H_2O$-ethanol, pH 7.0. Eluation is performed with NaCl-saturated 50% $H_2O$-ethanol, adjusting the pH value to 4.0 by addition of N hydrochloric acid. The ethanol is removed from the eluate by treatment in the rotary evaporator at 40°C, and (3) is taken up

into ethylacetate. After drying over magnesium sulphate the fraction is evaporated to an oil.

$^1$H-nmr. (90 MHz) $\delta$ 7.61 (NH, 1H), 7.34 (d, 1H), 7.26 (s, 1H), 7.14 (d, 1H) 6.78 (dd, 1H) 4.07 (q, J = 7.1 Hz, 2H), 3.67 (s, 2H) 3.30 (m, 2H), 2.45 (m, 2H), 1.18 (t, J = 7.1 Hz, 3H)
Mass Spectrum m/e 146 (base peak), 219, 261, 334.

The production of 5-(N-(3-sulphosuccinimidoxycarbonylethyl) carbamoyl)-oxy-indolyl-3-acetic acid ethylester, Na-salt of formula (4)

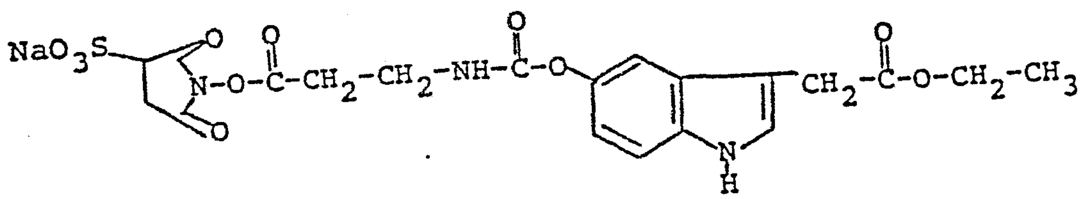

is performed by a carbodiimide condensation between (3) and the sodium salt of N-hydroxysulphosuccinimide as described by James V. Staros, Biochemistry, 1982, volume 21, p. 3950-3955.

Example 2

Preparation of a haptene-carrier conjugate with indole-3-acetic acid ethylester as ligand and a protein as carrier.

Bovine serum albumine (Cohn fraction V) is dissolved in 0.2 M potassium phosphate buffer, pH 7.4, concentration 100 mg/ml. Two portions of each

8 mg 5(N-(3-sulphosuccinimidoxy-carbonylethyl) carbamoyl)-oxy-indolyl-3-acetic acid ethylester, Na-salt are added to 100 $\mu$l of this solution with an interval of 2 hours. The reaction mixture is stirred for 16 hours at room temperature. Hereafter the reaction mixture is dialysed for 5 days at 4°C during stirring against 5 x 2 l 10 mM potassium phosphate, 0.15 M NaCl, pH 7.0, with daily changes of the outer fluid.

The finished indole-3-acetic acid ethylester conjugate is analyzed by UV-spectroscopy.

In the spectrum, cf. the subsequent Fig. 1 below, an indole shoulder with inflexion at 294-295 nm is observed.

In the subsequent FIG. 1 a UV-spectrum of bovine serum albumine (....) and indole-3-acetic acid conjugate (——) is shown at the bottom. At the top a difference spectrum of conjugate and carrier protein is shown.

The concentrations are adjusted at $\lambda_{max}$, so that A(278) = 0.45.

Example 3

Synthesis of 5-(N-(succinimidoxy-carbonyl-ethyl) carbamoyl)-oxy-indolyl-3-acetoxyacetate methylester of the formula (4)

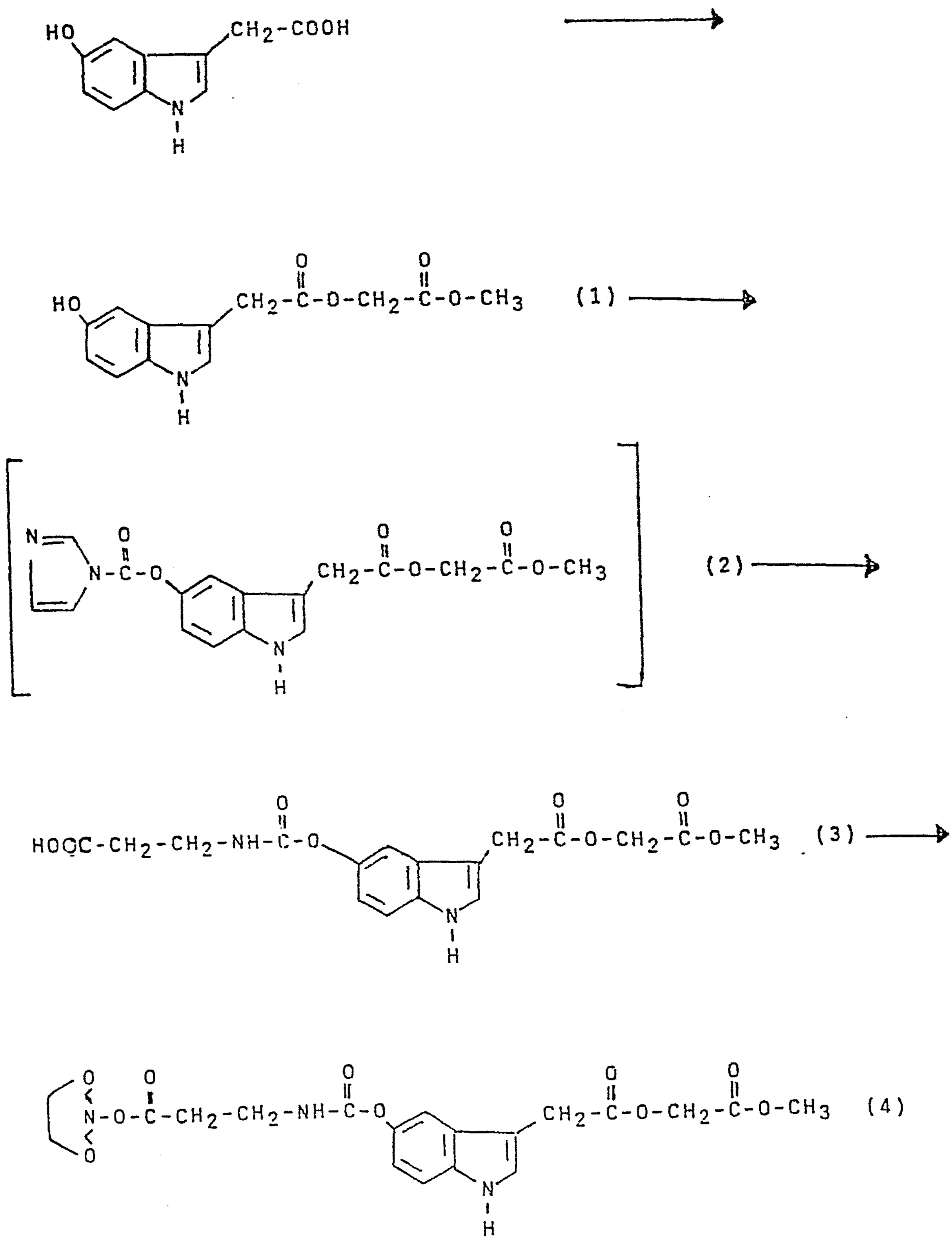

(1)

(2)

(3)

(4)

5-hydroxy-indolyl-3-acetoxy acetatemethylester of the formula (1)

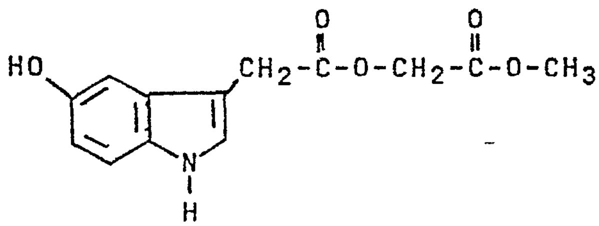 (1)

5 mmoles 5-hydroxy-indolyl-3-acetic acid are dissolved in 12 ml dry dimethyl sulphoxide (remaining water content < 0,03%), and 1 ml triethyl amine is added, and then the reaction container is closed under Ar. Under vigorous stirring at room temperature 6 mmoles bromo acetic acid methyl ester are added during 10 minutes. After finished addition the stirring is continued for additional 30 minutes at room temperature. Then the reaction mixture is diluted with 25 ml ethyl acetate and is washed with 4 x 50 ml 0,1 M $NaHCO_3$ followed by washing with 50 ml saturated aqueous NaCl and finally with 50 ml water. The organic phase is dried over water free $MgSO_4$, whereafter the seccative is filtered off. Ethyl acetate is removed in a rotary evaporator at 40°C, whereby the product crystallizes. The crystalline product is washed with cold ether.

$^1H$-nmr (90 MHz):
7.30-6.62 (m, 4H), 4.65 (s, 2H), 3.77 (d, J = 0.6Hz, 2H), 3.70 (s, 3H).

Mass spectrum m/e
263 M+ (15.7), 173 (9.8), 146 (100), 117 (5.3).

5-(N-(carboxy-ethyl) carbamoyl)-oxy-indolyl-3-acetoxyacetate methylester of the formula (3):

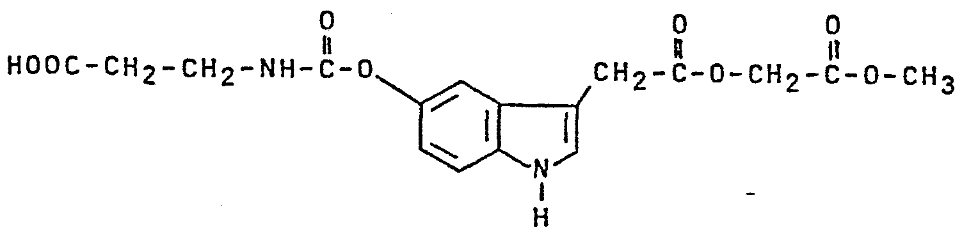

Mol relations and reaction conditions for the production of this compound are identical with those stated in example 1.

$^1$H-nmr (90 MHz):
7.45-6.72 (m), 4.66 (s), 3.85 (d, J = 0,61), 3.69 (s), 2.66 (q).

Quartet at about 3,4 cannot be seen because of water top.

Mass spectrum m/e:
378 M+ (0.08), 277 (1.8), 263 (22.3), 173 (13.7), 146 (100), 117 (6.6).

5-(N-(succinimidoxy-carbonylethyl) carbamoyl)-oxy-indolyl-3-acetoxyacetate methylester of the formula (4).

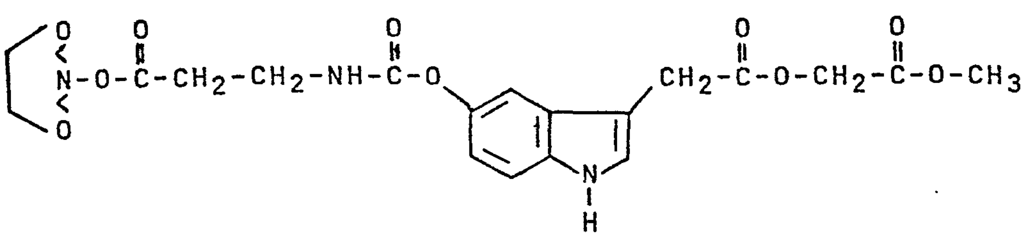

1 mmole (3) is dissolved together with 1 mmole N-hydroxysuccinimide in 2,5 ml dry peroxide free dioxane. Hereafter 1,2 mmole N,N'-dicyclohexyl carbodiimide is added. The reaction mixture is closed under Ar and is stirred for 12 hours at room temperature. The formed N,N'-dicyclohexyl urea carbamide is filtered off, and the dioxane is removed in a rotary evaporator at 40°C. The thus formed oil is taken up into 1,5 ml acetone and the last residue of N,N'-dicyclohexyl urea is removed by filtering. Acetone is removed in a rotary evaporator at 40°C. The evaporation residue containing (4) is used without further purification.

Example 4

Preparation of a haptene-carrier conjugate with indole-3-acetic acid as ligand and a protein as carrier

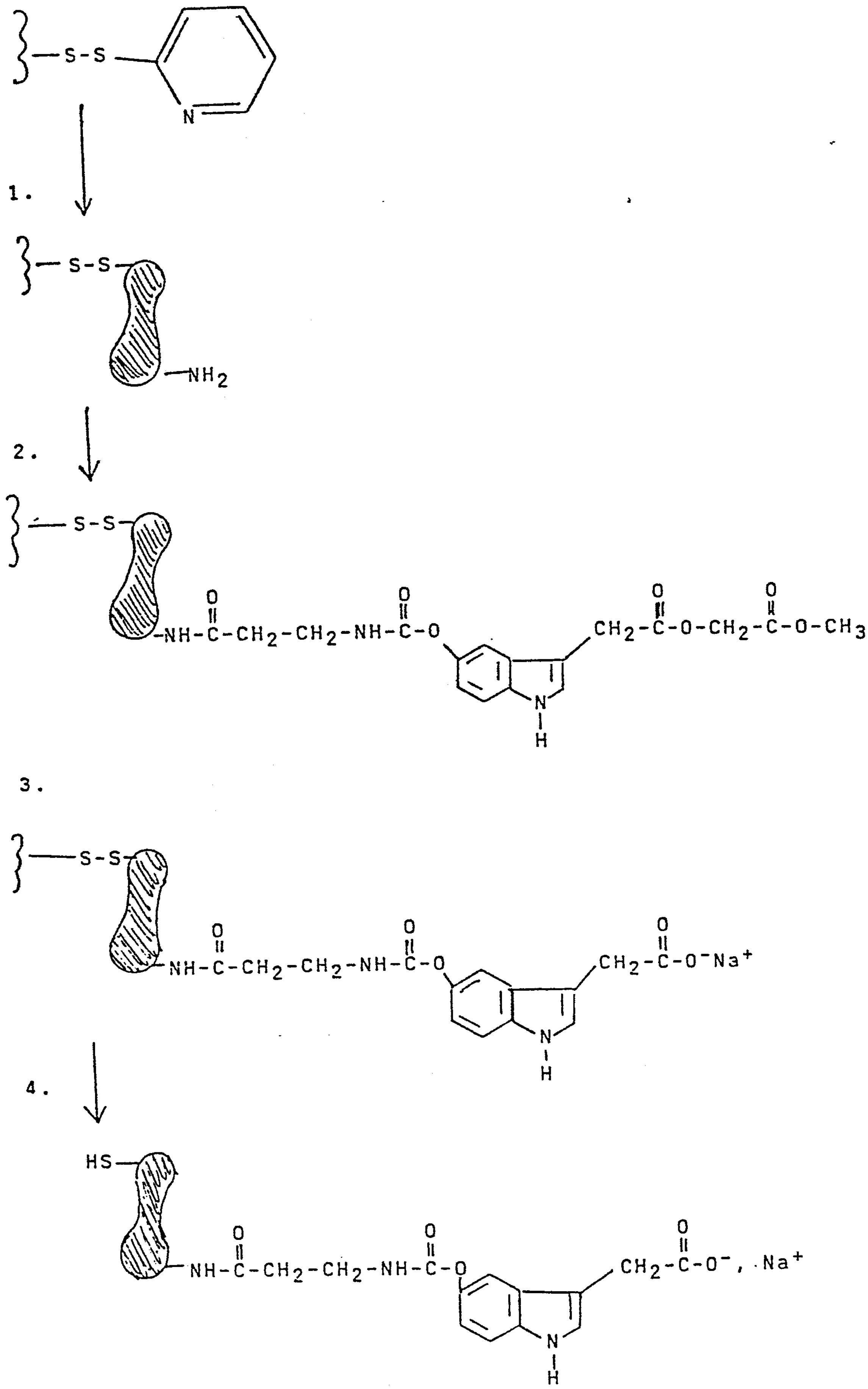
1.
2.
3.
4.

Step 1. Production of BSA Gel

0,5 g bovine serum albumine (Cohn fraction V) (BSA) is dissolved in 0,1 M $NaHCO_3$, 1 mM $Na_2EDTA$. The protein solution reacts over the night with 5 g Thiopropyl Sepharose$^R$ 6B (Pharmacia Fine Chemicals), which in advance has been washed with 0,1 M $NaHCO_3$, 1 mM $Na_2CO_3$. The reaction takes place under careful mechanical stirring at room temperature. Hereafter the gel is washed with 0,1 M $NaHCO_3$, 1 mM $Na_2EDTA$, until the wash water does no longer show absorption at 280 nm. Now there is washed with further 5 volumes of $H_2O$. Non-reacted glutathione-2-pyridyldisulphide groups are reduced by treating the washed gel with 20 ml 50 mM mercapto ethanol in 0,1 M acetic acid, pH-value 4,5. The gel is hereafter washed with 0,1 M acetic acid, pH-value 4,5 followed by washing with 0,1 M carbonate buffer, 1 mM $Na_2EDTA$, pH-value 8,0. In both cases a 10 times excess is used.

Step 2. Coupling of ligand to BSA Gel

5 g BSA gel is suspended in 50 ml 0,1 M carbonate buffer, 1 mM EDTA, pH-value 8,0. 0,1 mmole 5-(N-(succinimididoxy-carbonylethyl)carbamoyl)-oxy-indolyl-3-acetoxyacetate methylester is dissolved in 1 ml dimethyl formamide. Every half hour 0,2 ml of this solution is added to the gel suspension under mechanical stirring at room temperature. Half an hour after the final addition of ligand the gel is washed first with equilibration buffer and then with 50 mM borate buffer, and 1 mM $Na_2EDTA$, pH-value 8,6. For washing 100 ml of each buffer is used.

Step 3. Removal of the ester group of the ligand by treatment with esterase

The gel (2) is suspended in 50 ml 50 mM borate buffer, 1 mM $Na_2EDTA$, and 1300 U esterase (carboxylic ester hydrolase EC 3.1.1.1.) dissolved in 2 ml of the above buffer is added.
The reaction mixture is shaken for 48 hours.

Step 4. Reduction of disulphide bond between gel and haptene-carrier conjugate

The esterase treated gel is transferred to a column and is washed with 0,1 M sodium phosphate buffer, and 1 mM $Na_2EDTA$, pH-value 8,2, until the wash water is no longer showing absorption at 280 nm. Hereafter is washed with further 5 column volumes of the same buffer. The column is eluted with 50 mM mercapto ethanolal, 0,1 M sodium phosphate buffer, pH-value 8,2, until the eluate is no longer showing absorption at 280 nm. The eluate containing the indol-3-acetic acid-BSA conjugate, is dialyzed for 2 hours against running tap water followed by dialysis against 2 times 2 l 50 mM sodium phosphate buffer, pH-value 7,0.

On Fig. 2 is shown a UV-spectrum of this conjugate (A) as well as BSA (B). The protein concentration is in both cases 0,23 mg BSA per ml 0,1 M potassium phosphate buffer, pH-value 7,0 (protein determination according to Lowry's method).

Using $E^{1\%}_{280\ nm}$ = 10 as extinction coefficient

for BSA and $\varepsilon_{280}$ = 5200 $M^{-1}$ as the molar extinction coefficient for the ligand, a substitution degree of 34,4 moles of ligand per mol carrier-BSA can be calculated. The attention is especially drawn to the region 290-295 nm showing an inflexion as a sign of the fact that the extra UV-absorption originates from an indole structure.

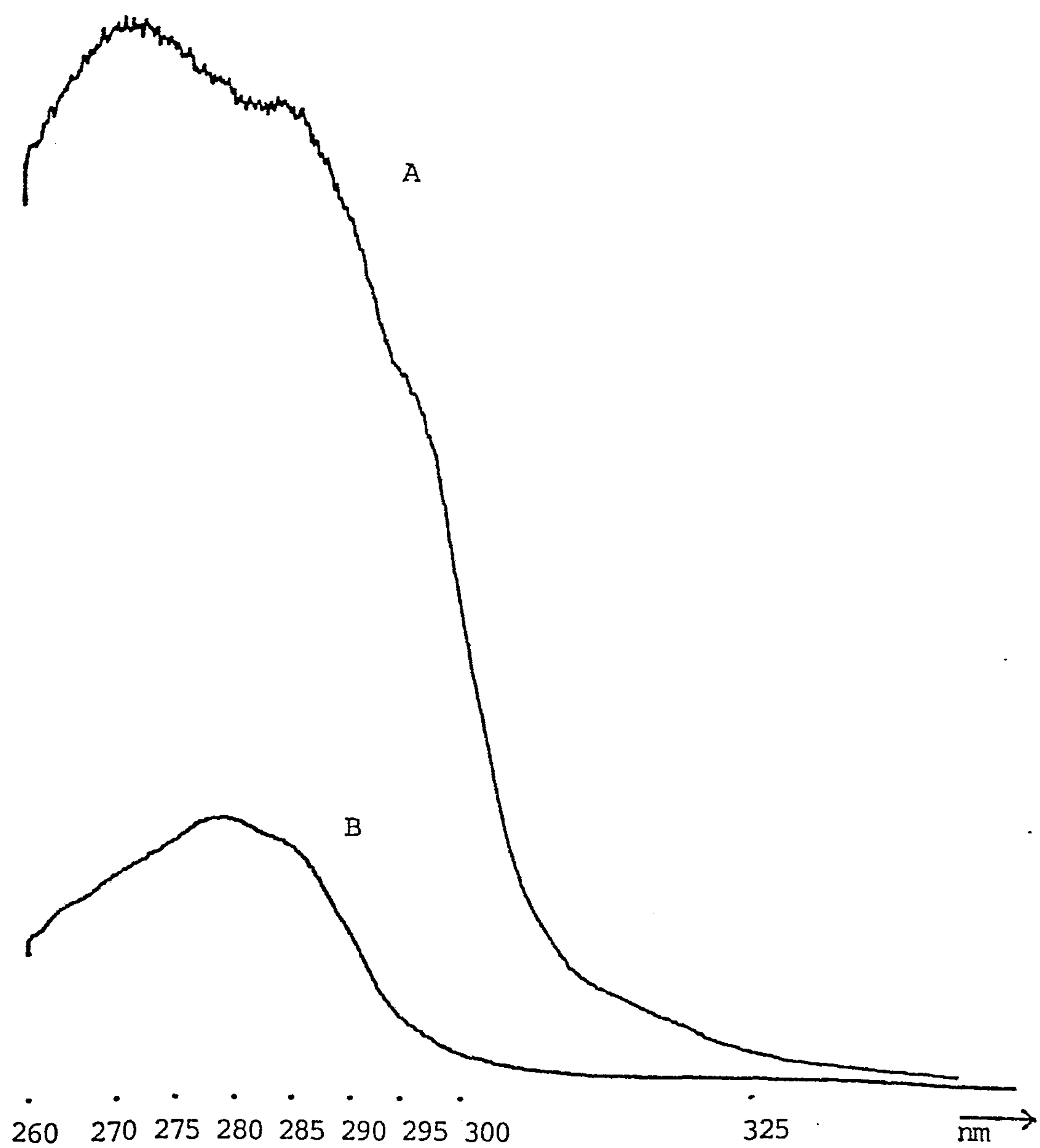
A
B
260
270
275
280
285
290
295
300
325
nm

Example 5

Synthesis of 5-(trifluorethan-sulfonyloxy-propane-3)-oxy-indolyl-3-acetoxyacetate methylester of the formula (4):

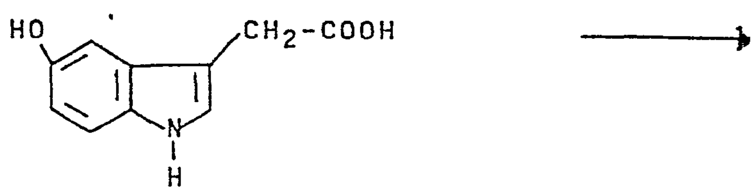

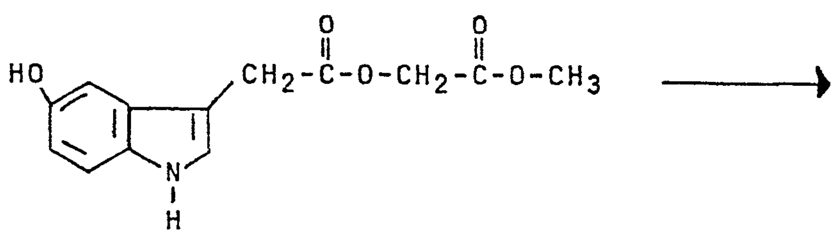

(1)

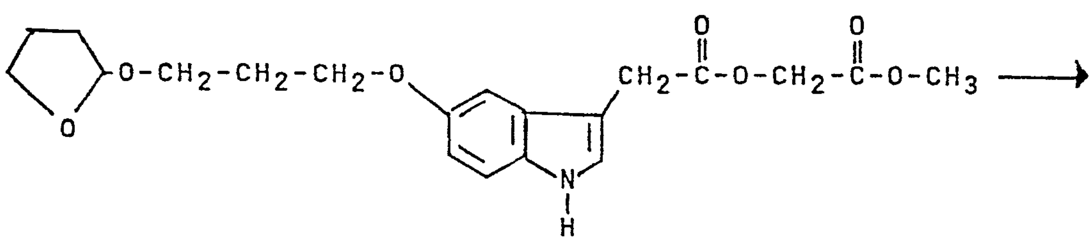

(2)

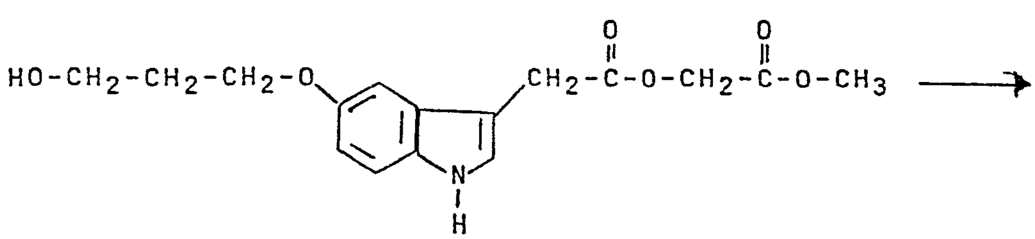

(3)

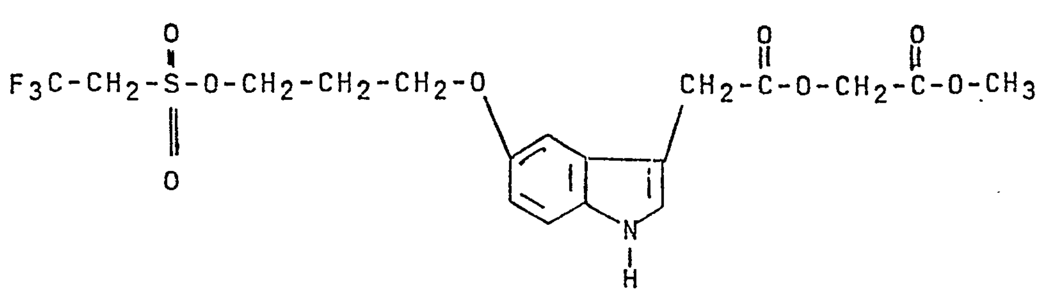

5-hydroxy-indolyl-3-acetoxyacetate methylester of the formula (1)

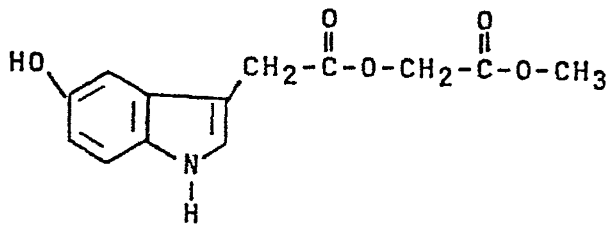

(1)

Synthesis of this compound is described in example 3.

5-(3-tetrahydrofuranyloxy-propane)-oxy-indolyl-3-acetoxyacetate methylester.

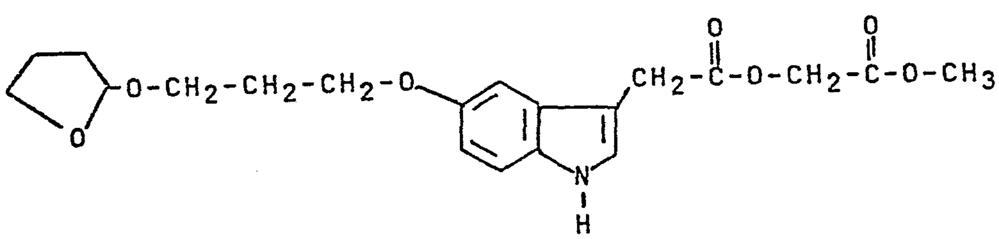

2 mmoles 5-hydroxy-indolyl-3-acetoxyacetate methylester are dissolved in 1 ml dry dimethyl sulphoxide (remaining water content < 0,03%). 7 ml 0,57 M tetraethyl ammonium fluoride dissolved in dry dimethylsulphoxide (remaining water content < 0,03%) are added simultaneously with 3 mmoles 3-bromo propane-1-tetrahydrofuranylether during 30 minutes to the 5-hydroxyindolyl-3-acetoxyacetate methylester solution under stirring in an Ar-atmosphere at room temperature. After finished addition the reaction mixture is stirred for further 30 minutes. The reaction mixture is extracted by three volumes of n-hexane followed by extraction by

five volumes of diethylether. The ether extracts are pooled and washed with three volumes 0,5 M sodium carbonate buffer, pH-value 12,0, followed by washing with $H_2O$ to neutral pH-value. After evaporation ofthe ether in a rotary evaporator at 30°C (2) is isolated together with a small amount of remaining water.

Mass spectrum m/e:
391 M+ (2.6), 321 (22.8), 303 (5.7), 289 (7.6), 263 (10.9), 262 (11.6), 204 (35.0), 146 (100), 117 (18.3), 70 (62.3).

5-(1-propanol)-oxy-indolyl-3-acetoxyacetate methylester of the formula (3).

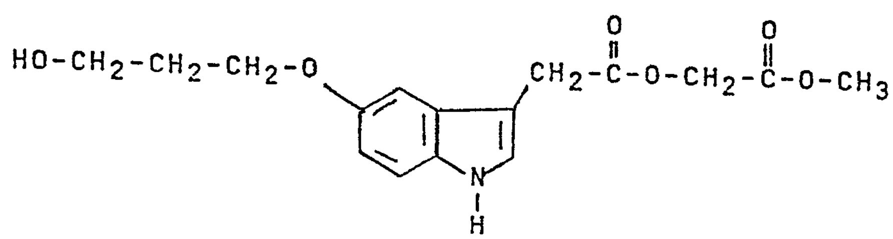

1,5 mmoles (2) is dissolved together with the present water residue in 10 ml methanol. The solution is closed under Ar, and 0,5 ml 2M HCl is added. After stirring for 1 hour 10 ml ethylacetate are added, and the reaction mixture is neutralized by addition of 10 ml 0,5 M $NaHCO_3$. The organic phase is isolated, washed with a volume of $H_2O$ and is dried over $MgSO_4$. The solvent is removed in a rotary evaporator at 40°C, whereafter (3) is isolated as an oil.

Mass spectrum m/e:
321 M+ (1.0), 263 (47.6), 204 (56.7), 146 (100), 117 (12.0).

5-(trifluorethane-sulfonyloxy-propane-3)-oxy-indolyl-3-acetoxyacetate methylester of the formula (4).

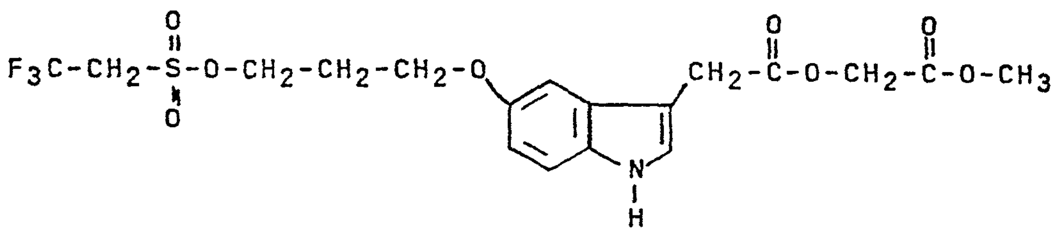

0,3 mmoles (3) is dissolved in 1 ml dry acetone (remaining water content < 0,01%), to which is added 0,4 ml dry pyridine (remaining water content < 0,01%). The solution is closed under Ar and is cooled to 0°C. 1,5 mmoles trifluorethansulphonylchloride dissolved in 5 ml dry acetone is added to the cooled solution during 10 minutes under vigorous stirring at 0°C. After stirring for further 10 minutes a complete transformation of (3) into (4) is observed.

Mass spectrum m/e:
467 (M+) (2.8), 350 (3.4), 339 (7.7), 303 (60.8), 262 (12.2), 186 (100).

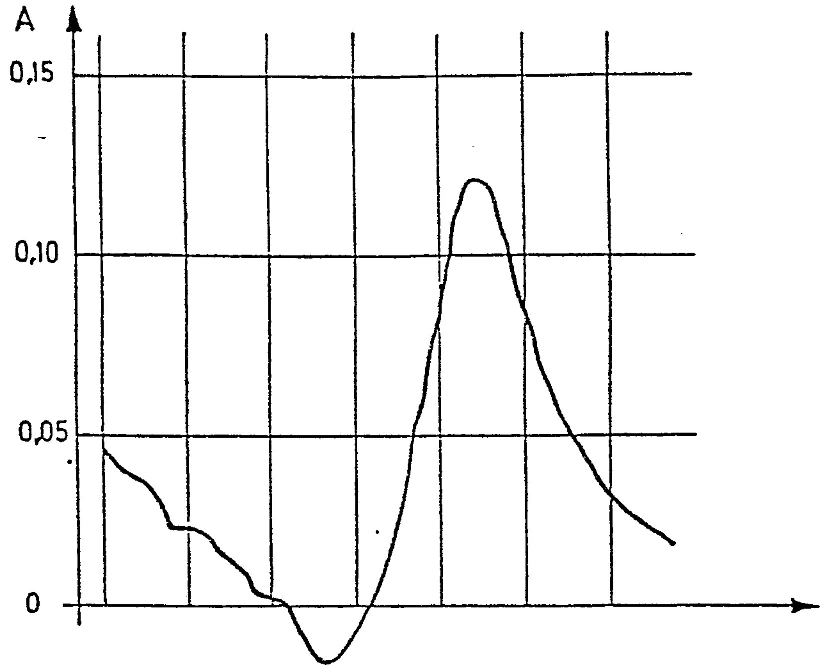


Fig. 1

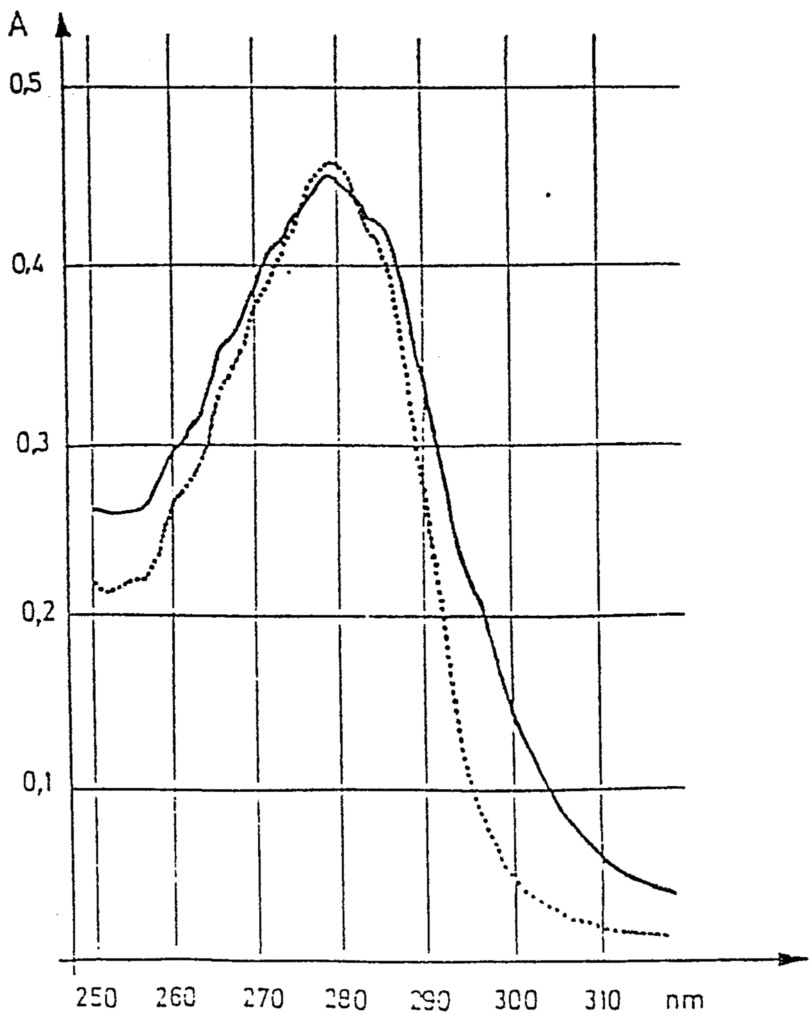

Claims:

1. A method of preparing indoles or indole derivatives coupled via position 4, 5, 6, or 7, characterised in reacting an indole compound being hydroxyl substituted in position 4, 5, 6, or 7 of the formula II

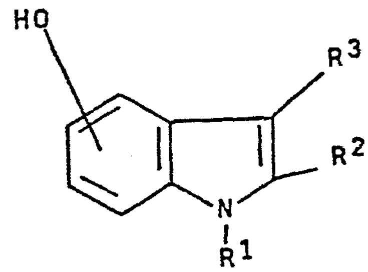

in which $R^1$, $R^2$, and $R^3$ each designates H or any other group not interfering with the subsequent steps, which groups may, if necessary, be protected by protective groups known per se, or 2 or all of the substituents $R^1$, $R^2$, and $R^3$ together with the atoms, to which they are connected, form a cyclic or heterocyclic system, with an activating reagent, and then or simultaneously introducing a nucleophilic group, optionally converting the resulting compound into an activated derivative thereof, such as an activated ester or a tosyl-, tresyl- or other derivative, which may be a part of a nucleophilic substitution reaction, or temporarily introducing this as a part of a leaving group to bind the indole hydroxyl group covalently to the functional group, which is to be coupled thereto, via a coupling residue.

2. The method according to claim 1 of preparing compounds of the general formula I:

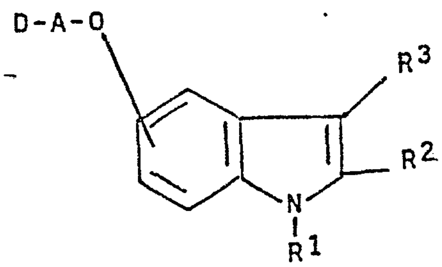

in which D is a group of the formula Y-X-D' containing a nucleophilic radical, in which X is a direct bond or a mono- or poly-substituted, straight chained or branched, saturated or unsaturated hydrocarbon group with 1-9 carbon atoms, D' is NH-, -S-, -O- or -O-Ph-, where Ph means phenyl, and Y is a carboxylic acid group or an activated ester group thereof, or Y is an alcohol group or a tosyl or tresyl derivative thereof or is temporarily introduced as a part of a leaving group and A is a coupling residue such as

$$\underset{\underset{O}{\|}}{-C-}\ ,\quad \underset{\underset{S}{\|}}{-C-},\quad -CH_2-\underset{\underset{OH}{|}}{CH}-CH_2-\quad \text{or}\quad -(CH_2)_m,$$

in which m is an integer from 1 to 9, and $R^1$, $R^2$, and $R^3$ have the meanings as stated in claim 1, characterised in reacting the indole structure of formula II as defined in claim 1 with a reagent, which provides the coupling residue A, and then or simultaneously introducing a nucleophilic group D, as defined above.

3. The method according to claims 1-2, characterised in that the activation is performed with a coupling reagent selected from the group consisting of carbonyl diimidazole, carbonyl thiodiimidazole or a epihalohydrine.

4. The method according to claims 1-2, c h a - r a c t e r i s e d in reacting the indole or the indole derivative with an activating reagent and then introducing a nucleophilic group by reaction with a nucleophilic reagent said reagent containing a thiol-, amino-, alcohol-, or phenol group.

5. The method according to claims 1-2, c h a - r a c t e r i s e d in reacting the indole or indole derivative with a substituted $C_{1-9}$-alkyl halogenid, whereby simultaneously a coupling residue A and a nucleophilic group D, in which A and D have the meanings mentioned in claim 2, are introduced.

6. The method according to claims 1-4, c h a - r a c t e r i s e d in that carbonyl diimidazole or carbonyl thiodiimidazole is used as coupling reagent, and that the activated indole structure is washed with a buffer at a pH-value below or about neutral pH.

7. The method according to claims 1-4 and 6, c h a r a c t e r i s e d in that a compound providing a carboxylic acid group is used as a nucleophilic reagent.

8. The method according to claim 7, c h a - r a c t e r i s e d in that an amino acid is used as a nucleophilic reagent.

9. The method according to claim 7, c h a - r a c t e r i s e d in that the carboxylic acid

group is converted into an activated ester.

10. The method according to claim 5, c h a - r a c t e r i s e d in reacting the indole or the indole derivative with 3-bromo propane-1-tetrahydrofuranylether.

11. Indoles or indole derivatives coupled via positions 4, 5, 6, or 7 c h a r a c t e r i s e d by the general formula I

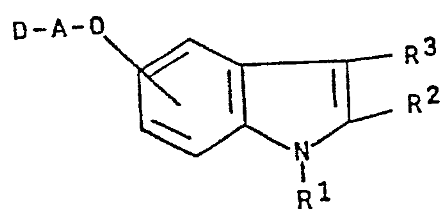

in which D-A-O is in position 4, 5, 6, or 7, and in which $R^1$, $R^2$, and $R^3$ each designates H or any other group not interfering with the subsequent steps, which groups are, if necessary, protected by protective groups known per se, or 2 or all of the substituents $R^1$, $R^2$, and $R^3$ together with the atoms, to which they are connected form a cyclic or heterocyclic system,

A is a coupling residue, and

D is a group of the formula Y-X-D' containing a nucleophilic radical, in which X is a direct bond or mono- or poly-substituted, straight chained or branched, saturated or unsaturated hydrocarbon group with 1-9 carbon atoms, D' is NH-, -S-, -O- or -O-Ph-, where Ph means phenyl, and Y is a carboxylic acid group or an activated ester group

thereof, or Y is an alcohol group or a tosyl or tresyl derivative thereof or is temporarily introduced as part of a leaving group.

12. Indoles or indole derivatives according to claim 11, c h a r a c t e r i s e d in that A is a group of the formula

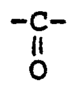 or 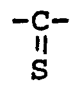

13. Indoles or indole derivatives according to claim 11, c h a r a c t e r i s e d in that A has the formula

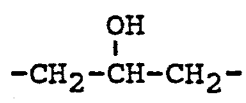

14. Indoles or indole derivatives according to claim 11, c h a r a c t e r i s e d in that A has the formula

$$(CH_2)_m$$

in which m is an integer from 1 to 9.

15. Indoles or indole derivatives according to claim 12, c h a r a c t e r i s e d by having the formula III

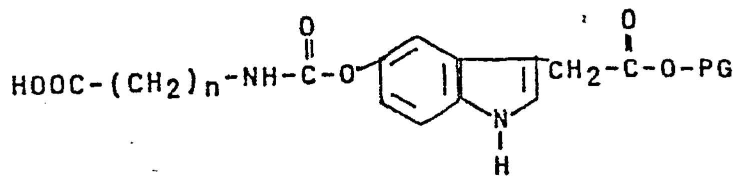

in which n is 0-9 and PG is a protective group.

16. Indoles or indole derivatives according to claim 14, c h a r a c t e r i s e d by having the formula IV

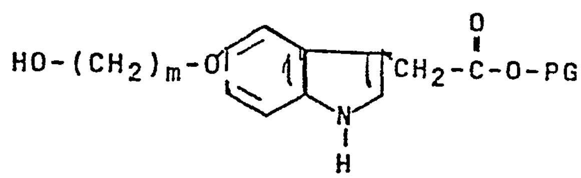

in which m has the meaning defined in claim 14 and PG has the meaning defined in claim 15.

17. Indoles or indole derivatives according to claim 12, c h a r a c t e r i s e d by having the formula V

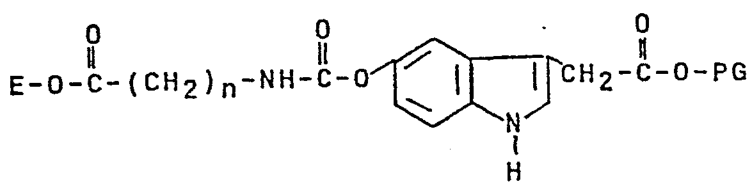

in which n and PG have the meanings indicated above, and -COOE is an activated ester group.

18. Indole derivative according to claim 15, c h a r a c t e r i z e d by having the formula VI

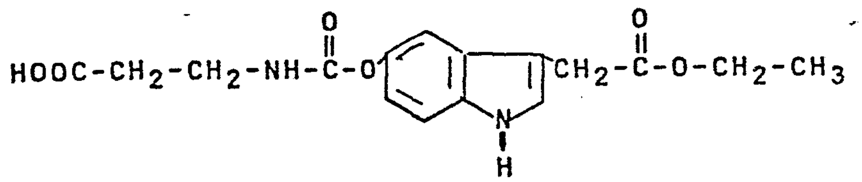

19. Indole derivative according to claim 17, c h a r a c t e r i z e d by having the formula VII

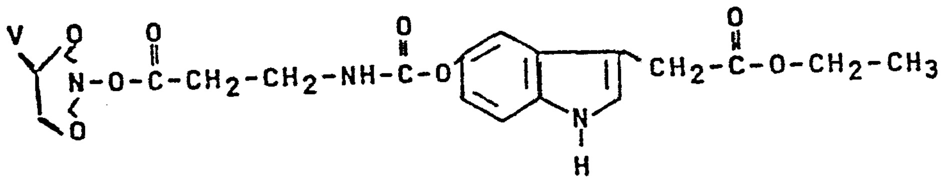

in which V is H or $-SO_3H$ or a salt thereof.

20. Indole derivative according to claim 14, c h a r a c t e r i z e d by having the formula VIII

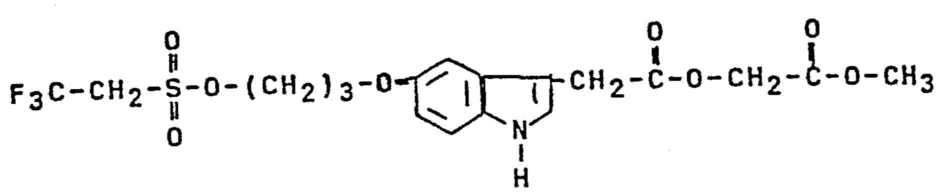

21. The use of a compound according to claim 11 in the preparation of biologically active indole-carrier-conjugates.

22. The use according to claim 21, wherein the carrier is a protein.

23. The use of a compound according to claim 18 or 19 in the preparation of biologically active affinity matrixes.

24. The use of a compound according to claims 18 or 20 in the preparation of biologically active affinity matrixes.